# EUROPEAN PATENT APPLICATION

(11) **EP 2 743 353 A1**
(43) Date of publication of application: **18.06.2014**
(21) Application number: 12197060.2
(22) Date of filing: 13.12.2012
(51) Int. Cl.: C12Q 1/68, B01J 19/00

(54) **Interstitial amplification**

(71) Applicant: Alacris Theranostics GmbH, 14195 Berlin (DE)
(72) Inventor: Glökler, Jörn, Dr., 10783 Berlin (DE); Mertes, Florian, Dr., 14195 Berlin (DE)
(74) Representative: Fanelli Haag Kilger PLLC

(57) **Abstract**

Herein disclosed is a nucleic acid amplification method comprising the steps of (a) providing a support comprising a plurality of interconnected first cavities; (b) contacting said support with reagents for carrying out an amplification reaction; (c) contacting said support with a sample of nucleic acids; and (d) carrying out the nucleic acid amplification reaction in said first cavities; wherein all or at least a fraction of the interconnections reduce or prevent a convective flow between the first cavities during amplification. Further disclosed are a support, a kit and their use for carrying out said method.

## Description

### FIELD OF THE INVENTION

The present invention is in the field of biology and diagnostics. It is more particularly in the field of molecular biology and in vitro diagnostics and more specifically relates to nucleic acid amplification.

### INTRODUCTION

Genotyping assays often require substantial amounts of DNA. To overcome the problem of limiting amounts of available DNA, Whole Genome Amplification (WGA) methods have been developed.

WGA is a method that amplifies small amounts of genomic DNA several thousand-fold in vitro. Several WGA methods based on the polymerase chain reaction (PCR) with Taq polymerase were initially developed. These methods included the use of primers directed at highly repetitive sequences, ligation of linkers to fragmented DNA, degenerate oligonucleotide primed PCR, and primer extension preamplification. All these methods suffer from a relatively high level of mutations in the amplified DNA (error rate 3x10-5) and highly non-uniform amplification due to the low fidelity and low processivity of the Taq polymerase, respectively.

Taking advantage of the high processivity and low error rate of the phi 29 bacteriophage DNA polymerase the multiple displacement amplification (MDA) method has become the preferred choice. The MDA process is isothermal and uses random primers to target the entire genome. The polymerase has strong strand displacement activity so that exponential amplification occurs through a branching mechanism, resulting in a high yield of DNA. MDA-based WGA has been frequently used in DNA sample preparation for genotyping and sequencing in recent years. For example, short tandem repeats (STR) and Human Leucocyte Antigen typing were performed using DNA amplified by MDA from a single cell. MDA-WGA has also been increasingly used in the field of forensic testing.

The WGA process, however, often result in non-uniform amplification of the DNA in which some regions of the genome are over-represented and others are under-represented. Additionally, strong background formation due to a preferred amplification of short sequences is a regularly occurring problem. Such biased amplification could make the WGA DNA unsuitable for studies where a uniform amplification is of concern, e.g. if the DNA produced by WGA is to be sequenced.

Thus, in massive parallel sequencing technology clonal amplification has become the state of the art. Clonal amplification led to a significant reduction of background amplification, thus, straightening the subsequent analysis of sequences.

Different clonal amplification methods have been developed in recent years. For example, emulsion PCR isolates individual DNA molecules along with primer-coated beads in aqueous bubbles within an oil phase. Polymerase chain reaction (PCR) then coats each bead with clonal copies of the DNA molecule followed by immobilization for later sequencing. Emulsion PCR is used in the methods by 454 Life Sciences, polony sequencing and SOLiD sequencing. In some applications, amplification is performed in emulsion bubbles in the absence of any beads. Another method for in vitro clonal amplification is bridge PCR, where fragments are amplified upon primers attached to a solid surface.

Current methods, however, suffer from a number of disadvantages. The use of an emulsion requires additional chemicals, mixing in order to form an emulsion, and, more significantly, a subsequent recovering step in order to remove the oil phase from the water phase. Further, the preparation of primer bearing beads or surfaces is cumbersome and expensive. Likely the greatest difficulty of using emulsions is the stability of the micelles during the amplification process, and the often denaturing conditions under which they are formed and broken down. Despite recent improvements, the user needs to handle viscous fluids and larger volumes of organic solvents which hinder a greater parallelization and automation of the process.

Alternative, non-preparative methods that generate clonal amplification clusters or polonies are based on a simple separation in a homogeneous matrix (Chetverin et al., 1997. Method for amplification of nucleic acids in solid media), covalent immobilization to a surface (Kawashima et al. 1998. Method of Nucleic Acid Amplification; Mitra et al. 1999. In situ localized amplification and contact replication of many individual DNA molecules. Nucleic Acids Res. 27, e34; Shi et al. 2000. Covalent attachment of unmodified nucleic acids to silanized solid phase surfaces) or by a separation into different chambers (Brown et al. 2000. Method of sampling, amplifying and quantifying segment of nucleic acid, polymerase chain reaction assembly having nanoliter-sized sample chambers, and method of filling assembly). These can be embedded in a gel matrix, capillaries and open chambers generated by microfabrication. Retrieval of amplified nucleic acid pools from the matrix or device is not intended or even possible. Such polonies or clusters are spread in two dimensions and mostly applied to massive parallel assays such as digital PCR or next generation sequencing methods (Kawashima et al. 1998; Shendure et al. 2005. Accurate multiplex polony sequencing of an evolved bacterial genome. Science 309, 1728-1732).

Due to the above drawbacks of the present methods, it would be desirable to have a method that does not have said drawbacks. This problem is solved by the present invention.

### BRIEF DESCRIPTION OF THE INVENTION

The problem is solved by the provision of a nucleic acid amplification method comprising the steps of (a) providing a support comprising a plurality of interconnected first cavities; (b) contacting said support with reagents for carrying out an amplification reaction; (c) contacting said support with a sample of nucleic acids; and (d) carrying out the nucleic acid amplification reaction in said first cavities; wherein all or at least a fraction of the interconnections reduce or prevent a convective flow between the first cavities during amplification.

The invention also relates to a support comprising a plurality of interconnected first cavities, wherein all or at least a fraction of the interconnections reduce or prevent a convective flow between the first cavities during an nucleic acid amplification reaction, wherein the support further comprises a plurality of second cavities communicating with the first cavities, and wherein the second cavities serve as a repository for and comprise reagents for carrying out an amplification reaction.

The invention further relates to a kit comprising a support as defined herein, a polymerase, and optionally the following: nucleotides and/or primers.

Also encompassed by the present invention is the use of a support as defined herein or use of a kit as described herein for nucleic acid amplification, preferably, for whole genome amplification.

### Definitions

The term "porous" herein refers to pores in which fluid can enter including communicating and dead-end pores. Such pores may be also denoted as microchannels and/or microcavities.

The term "cavity" can be interchangeable used with the term pore. It is to be noted, however, that herein it is discriminated between first cavities and second cavities. First cavities are those in which an amplification reaction takes place. Second cavities are provided in a preferred embodiment. They may be seen as a reservoir for providing at least some of the reagents for carrying out the amplification reaction, such as primers and/or nucleotides. In a further preferred embodiment, the second cavities are not capable of accommodating enzymes, e.g. a polymerase, and/or the nucleic acid to be amplified due to size restrictions.

A "sample of nucleic acids" herein denotes a sample which comprises at least one, preferably at least 2, 3, 4, 5, 6, 7, 8, 9, 10, 10², 10³, 10⁴, 10⁵, 10⁶, 10⁷, 10⁸, or 10⁹ nucleic acids. The nucleic acids preferably differ in size and/or sequence. The sample may be obtained from any source and include plasmids and cloned nucleic acids, nucleic acids from any source, including viruses, bacteria, archaea, and eukaryotic organisms, e.g. yeast, molds, algae, plants or animals. Nucleic acids may be extracted from, for example, blood or other fluid, or tissue material such as chorionic villi or amniotic cells by a variety of techniques such as that described by Maniatis et al., Molecular Cloning: A Laboratory Manual, (New York: Cold Spring Harbor Laboratory) pp 280-281 (1982). Preferably, the sample includes substantially an entire genome. The genome may be ideally fragmented prior to contacting it with the support.

The term "nucleic acid" is here used in its broadest sense and comprises ribonucleic acids (RNA) and deoxyribonucleic acids (DNA) from all possible sources, in all lengths and configurations, such as double-stranded, single-stranded, circular, linear or branched. All sub-units and sub-types are also comprised, such as monomeric nucleotides, oligomers, plasmids, viral and bacterial nucleic acids, as well as genomic and non-genomic DNA and RNA from animal and plant cells or other eukaryotes or prokaryotes, mRNA (messenger RNA) in processed and unprocessed form, tRNA (transfer RNA), hn-RNA (heterogeneous nuclear RNA), rRNA (ribosomal RNA), LNA (locked nucleic acid), mtRNA (mitochondrial), nRNA (nuclear RNA), siRNA (short interfering RNA), snRNA (small nuclear RNA), snoRNA (small nucleolar RNA), scaRNA (Small Cajal Body specific RNA), microRNA, dsRNA (double-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or as well as all other conceivable nucleic acids.

The term "primers" herein may denote one or more sets of target-specific primers, each set comprising a forward and a reverse primer, random primers, degenerate primers, or random exonuclease-resistant primers depending on the number of nucleic acids to be amplified and the amplification method used. Primers herein also refer to low melting point primers, which usually have a length between 4 and 10 nucleotides with on average 6 nucleotides in length. The term "primers" also comprises a reaction mixture of nucleotides and a primase, which may be used to synthesize primers. Preferred primers have a length of about 10 - 100, more preferably about 15 - 50, most preferably about 18 - 40 bases. In some cases, preferred primers have a length of about 4 to 10, preferably about 5 to 8, most preferably about 6 bases.

Primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing primers on a modified solid support is described in U.S. Pat. No. 4,458,006, which is hereby incorporated by reference. It is also possible to use a primer which has been isolated from a biological source (such as a restriction endonuclease digest).

The term "polymerase" may refer to any enzyme with a replication activity which can be used in one of the amplification methods described herein.

Some amplification methods make use of a "helicase". Helicases are known by those skilled in the art. They are proteins that move directionally along a nucleic acid phosphodiester backbone, separating two annealed nucleic acid strands (e.g. DNA, RNA, or RNA-DNA hybrid) using energy derived from hydrolysis of NTPs or dNTPs. Based on the presence of defined helicase motifs, it is possible to attribute a helicase activity to a given protein. The skilled artisan is able to select suited enzymes with helicase activity for the use in a method according to the present invention. In a preferred embodiment the helicase is selected from the group comprising helicases from different families: superfamily I helicases (e.g. dda, pcrA, F-plasmid tral protein helicase, uvrD), superfamily II helicases (e.g. recQ, NS3-helicase), superfamily III helicases (e.g. AAV rep Helicase), helicases from DnaB-like superfamily (e.g. T7 phage helicase) or helicases from Rho-like superfamily.

As used herein, the term "nucleotides" refers to ribonucleoside triphosphates and deoxyribonucleoside triphosphates. Non-limiting examples of such deoxyribonucleoside triphosphates (dNTPs) are dATP, dGTP, dCTP, dTTP, dUTP, which may also be present in the form of labelled derivatives, for instance comprising a fluorescent label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Non-limiting examples of such ribonucleoside triphosphates (NTPs) are ATP, GTP, CTP, TTP, UTP, which may also be present in the form of labelled derivatives, for instance comprising a fluorescent label, a radioactive label, a biotin label. As used herein, the term nucleotide refers to deoxyribonucleoside triphosphates. Non-limiting examples of such dNTPs are dATP, dGTP, dCTP, dTTP, dUTP, which may also be present in the form of labelled derivatives, for instance comprising a fluorescence label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Furthermore, ddNTPS of the above-described molecules are encompassed in the present invention.

### DETAILED DESCRIPTION OF THE INVENTION

The inventors found that, in order to prevent contaminations, artefacts, and bias, miniaturized highly parallel amplification reactions can be performed in cavities provided, for example, by densely packed spherical beads. This effect is considered to be the result of a distribution of the nucleic acids to be amplified in individual cavities, thereby forming micro amplification vessels. Such micro reaction vessels can be formed by, for example, a pore system in a support, such as a monolithic column comprising microchannels through said column or a sponge-like solid, or by the interstitial void in a support formed by beads.

### Nucleic acid amplification method

The invention, thus, relates to a nucleic acid amplification method comprising the steps of providing a support comprising a plurality of interconnected first cavities; contacting said support with reagents for carrying out an amplification reaction; contacting said support with a sample of nucleic acids; and carrying out the nucleic acid amplification reaction in said first cavities; wherein all or at least a fraction of the interconnections reduce or prevent a convective flow between the first cavities during amplification.

### Supports suitable for the nucleic acid amplification method

The present method is assumed to be based on the distribution of nucleic acids in interconnected micro amplification vessels (first cavities). As such, any support is conceivable provided that it comprises first cavities being interconnected and, thus, enabling mass transport. The interconnections, however, must also act as a barrier for convective transport between individual first cavities. A barrier does not need to prevent any convective transport. It is sufficient that it reduces convective transport, particularly, during amplification reactions which most often employ a temperature above ambient. As a result, during amplification, the amplicons stemming from a first nucleic acid remain within the same cavity and do not disturb the amplification of a second nucleic acid present in another cavity.

The support is preferably a solid or a gel. The support preferably does not form a homogeneous matrix when it is contacted with an aqueous solution such as a buffer, sample, reagents for carrying out an amplification reaction and the like.

Preferably, the support is made of a porous material or a plurality of beads or a combination thereof. Apparently, the pore system in the porous material will provide the first cavities as described herein. If beads are used, the interstitial void between the beads will define said first cavities. Conveniently, the beads are arranged such that at least a fraction of the interconnections reduce or prevent a convective flow between the first cavities during amplification. This may be achieved by densely packing said beads. It is further preferred that the beads are spherical. However, to put the present method into practice neither ideally spherical nor almost spherical particles are required. This means, also angular beads or randomly shaped beads are possible as long as they provide first cavities as described herein.

Without being intended to be limited to a particular support, the combination of a porous material and a plurality of beads according to a preferred embodiment may resemble a size exclusion chromatography resin. Such resin usually comprises small porous beads. The pore system in the beads offers relatively small compounds to be purified or separated to penetrate into microchannels and/or microcavities, thus eluting late. Compounds which are too big to fit into the pore system will remain in the so-called interstitial void, i.e. the space between the beads, not be retained and, thus elute early. The principle of purification is well-known in the art and does not need further explanation. In the present method, however, the pore system within the beads will ideally serve as a repository for at least some of the reagents for carrying out an amplification reaction (see above). In the cavities between the beads (interstitial void), the amplification reaction is carried out. As the person skilled in the art is aware of, the above described embodiment is not limited to a support resembling a size exclusion chromatography resin as long as a support is provided comprising first and second cavities as defined herein.

The person skilled in the art will know a variety of different supports being offered for size exclusion chromatography, all of which may be used in the context of the present disclosure. Likewise, methods of producing particle-like supports are well-known. He will further be aware that some supports are suited for separating molecules of a particular size, while some supports are suited for separating molecules of another size depending on the diameter of the pores and/or the diameter of the particles. Though the present method does not relate to the separation of molecules by size, the aforementioned dimensions may be selected in dependency of the polymerase used. For example, the pore system of the support may exclude the polymerase used, preferably molecules (e.g. proteins) bigger than 50 kDa, more preferably bigger than 30 kDa. Suitable and commercially available supports are, for example, Sephadex G-10 to G-75, Superdex-30 to 75 and Toyopearl 30 to 50.

Advantageously, porous materials can make the beads permeable for low-molecular weight compounds, such as buffer components, nucleotides and primers. These porous beads can act as repository for nucleotides and primers and supply more fuel to the interspaces as the reaction continues. However, it is preferred that template nucleic acid and polymerase remain in the space formed between the beads (interstitial void).

More generally, the above embodiment is reflected by a support as described herein that further comprises a plurality of second cavities communicating with the first cavities, and wherein the second cavities serve as a repository for at least some of the reagents for carrying out an amplification reaction. This means, the support which is made of porous beads, most preferably comprises first cavities that are formed by the interstitial void between the beads, and second cavities that are formed by the pore system within the beads.

In order to reduce or at least prevent a convective flow between all or at least a fraction of the first cavities during amplification, the interconnections may act as a physical barrier. In the case of beads cavities formed between the beads are separated by narrow passages that allow only a slow interchange of substances mainly by diffusion. This means, only small molecules (not nucleic acids to be amplified) are expected to pass from one cavity to another by diffusion because diffusive velocity of small molecules is bigger than that of big molecules. For example, the interconnections between the first cavities may be limited to a diameter between 1 µm to 500 µm, preferably 2 µm to 200 µm, most preferably 5 µm to 100 µm. Alternatively, the barrier may be modulated by selecting beads of different diameters. For example, the beads on average may have a diameter of 1 µm to 1000 µm, preferably 2 µm to 200 µm, most preferably 5 µm to 100 µm. These values shall be understood as average values. The person skilled in the art will appreciate that such strict restriction does not need to apply to all interconnections. Rather, it is enough that a fraction of the interconnections provide a barrier. Also, a barrier must not be present over the full length of an interconnection.

In order to create further reaction chambers a support may be provided comprising a plurality of first beads and a plurality of second beads, wherein first and second beads differ in size (diameter). Ideally, second beads will arrange within the interstitial void of the first beads, thus creating more than one micro reaction chambers within the space formed by first beads. For example, the first beads have a diameter as described below and the second beads have a smaller diameter, e.g. on average 0.1 µm to 500 µm, preferably 0.2 µm to 100 µm, more preferably 0.5 µm to 50 µm, most preferably 0.5 µm to 30 µm. It is further preferred that the density of the first and second beads are within the same order of magnitude, more preferred is that the density is substantially identical.

Optionally, the support does not substantially interact with the nucleic acids.

The support may be made of glass, magnetite, polystyrene, an acrylate or a polysaccharide, preferably polyacrylamide, polymethacrylate, agarose, sepharose and dextran.

If the support comprises beads their diameter may be on average 1 µm to 1000 µm, preferably 2 µm to 200 µm, more preferably 5 µm to 100 µm, most preferably 10 µm to 60 µm. It is to be noted, however, the support is not restricted to these dimensions provided that a convective flow between the first cavities is reduced or prevented (see above).

In one embodiment the support is contained within a vessel having at least a first opening through which reagents and sample may be added and/or withdrawn. The vessel may comprise a second opening, e.g. for through-flow operation. In some cases it might be convenient if the first and/or the second opening comprise a filter or a mesh which retains the support.

Further, the support may be contained in a column, such as a chromatography or spin-column, having openings at the top and the bottom, and wherein the reagents and the sample is contacted with the support by loading said reagents and said sample, respectively, onto said column via the top or bottom opening. In some cases it might be convenient if the openings comprise a filter or a mesh which retains the support.

If beads of the same size are used as a support, the thus contained beads can be assumed to be monodisperse, however, the packing is to be considered best described by a random close pack. Both soft and solid beads can be used to form the cavities. Hence, the exact number of beads to be filled in the column and the resulting volume of the support may vary depending on material, buffer and as the case may be (centrifugal) force applied to the column.

### The steps involved in the nucleic acid amplification method

The herein disclosed method involves bringing into contact the support with a solution comprising reagents for carrying out an amplification reaction and bringing into contact the support with a sample of nucleic acids.

Contacting the support with reagents/sample is to be understood in its broadest sense, i.e. part or, preferably, substantially all of the support is brought into contact such that the reagents/sample are distributed within the pore system of the support. Preferably, the reagents and/or the sample are evenly distributed within the support. In most cases, it should be ensured that the reagents and the sample are contained or retained within the support until amplification has finished. This may be achieved by physical forces between the reagents/sample, which are usually present in an aqueous solution, and the support, such as capillary forces and surface tension.

Reagents for carrying out an amplification reaction depend on the particular amplification reaction to be carried out. For example, if the amplification reaction is a PCR, these reagents will include at least primers, a polymerase, and nucleotides. If the amplification reaction is a HDA reaction, the reagents will additionally include a helicase. Common to all methods the reagents will, accordingly, include a polymerase, either primers or a primase, and nucleotides.

The support may be contacted with the reagents before it is contacted with the sample, or vice versa. Alternatively, the reagents are split, whereof some are added before and some are added after the sample. For example, the support may be first contacted with nucleotides and primers. Then, the support is contacted with the sample. Afterwards, the support is contacted with a polymerase (and, as the case may be, a helicase). The above example considers the fact that small molecules such as nucleotides and primers will be more efficiently retained within the support than bigger molecules such as an enzyme. Thus, if small molecules are added at the very beginning, they will probably be more evenly distributed than if they are added in later steps. However, it is also possible to perform the steps of contacting the support with some or all reagents for carrying out an amplification reaction and contacting the support with a sample of nucleic acids not only sequentially but also in parallel.

Contacting preferably means equilibration. Equilibration is understood to determine a stable and equal distribution of reagents in the pore system and void volume of the support. To achieve a stable and equal distribution, the solution comprising the respective reagents are preferably support repeatedly exchanged with an equal solution.

Nucleic acid amplification, in the method described herein, is performed directly on said support, i.e. in the first cavities where the nucleic acids to be amplified are distributed. For this purpose, the support is to be exposed to the temperature scheme which is required for the respective amplification method. While the previous contacting steps may have involved a flow through the support, amplification is conducted under non-flowing conditions. Amplification is continued until a sufficient number of amplicons have been generated.

Preferably, a single cavity comprises only a limited number of different nucleic acid to be amplified (i.e. template). A limited number ideally means a single nucleic acid, but may also mean 2, 3, 4, 5, 6, 7, 8, 9, 10, or more nucleic acids differing in length and/r sequence.

The herein disclosed principle is versatile and not restricted to a particular amplification method. That means, nucleic-acid amplification can be accomplished by any of the various nucleic-acid amplification methods known in the art, including but not limited to the polymerase chain reaction (PCR), real-time PCR (rtPCR), helicase-dependent amplification (HDA), recombinase-polymerase amplification (RPA), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification. In case of Qb amplification, there are neither primers nor a primase necessary.

In some cases, it might be convenient that the amplification method is an isothermal method. In other cases, a non-isothermal method may be preferred. By "isothermal amplification reaction" it is meant that the temperature does not significantly change during the reaction. In a preferred embodiment the temperature of the isothermal amplification reaction does not deviate by more than 10 °C, preferably by not more than 5 °C, even more preferably not more than 2 °C during the main enzymatic reaction step where amplification takes place. The isothermal amplification method may be selected from the group of helicase dependent amplification (HDA) (Vincent et al. (2004) EMBO rep 5(8):795-800), thermostable HDA (tHDA) (An et al. (2005) J Biol Chem 280(32):28952-28958), recombinase polymerase amplification (RPA) (Piepenburg et al. (2006) PloS Biol 4(7):1115-1120),loop-mediated isothermal amplification (LAMP), SMAP (smart amplification process) and the like. The non-isothermal amplification method may be selected from the group of polymerase chain reaction (PCR) (Saiki et al. (1985) Science 230:1350), quantitative real-time PCR (rtPCR). Depending on the method of isothermal amplification of nucleic acids different enzymes are required for the amplification reaction. Known isothermal methods for amplification of nucleic acids are the above mentioned, wherein the at least one mesophilic enzyme for amplifying nucleic acids under isothermal conditions is selected from the group consisting of helicase, mesophilic polymerases, mesophilic polymerases having strand displacement activity, recombination proteins.
The method and the support described herein is particular useful if a high number of different nucleic acids are to be amplified in parallel. Hence, it is preferred that the amplification reaction is a whole genome amplification method. Thus, it is preferred that the amplification method is a whole genome amplification (WGA) method. PCR-based methods for WGA include degenerate oligonucleotide primed PCR (DOP-PCR; Cheung and Nelson 1996, Proc. Natl. Acad. Sci. 93:14676-79) and primer extension PCR (PEP; Zhang et al. 1992, Proc. Natl. Acad. Sci. 89:5847-51). WGA may be further achieved by multiple-displacement amplification (MDA), which uses the highly processive ϕ29 DNA polymerase and random exonuclease-resistant primers in an isothermal amplification reaction (Dean et al. 2002, Proc. Natl. Acad. Sci. 99:5261-66). Another WGA method was commercialized by Rubicon Genomics as OmniPlex. OmniPlex converts randomly fragmented genomic DNA into a library of inherently amplifiable DNA fragments of defined size. This library can be effectively amplified several thousandfold with the help of a high-fidelity DNA polymerase. The library can be re-amplified to achieve a final amplification of over a millionfold without degradation of representation (Langmore 2002, Pharmacogenomics 3:557-60).

The amplification methods will require buffers, nucleotides in addition to the enzymes required. Hence, in the context of the present method, the reagents for carrying out an amplification reaction comprise at least a polymerase, nucleotides and one or more primers, or a polymerase, nucleotides and a primase.

Conveniently, the primers are selected from the group of random primers, degenerate primers and random exonuclease-resistant primers, and/or wherein the polymerase is selected from the group of Phi 29 polymerase depending on the type of nucleic acid amplification method used.

The sample of nucleic acids may comprise nucleic acids fragments. Fragments have preferably a length of 0.1 kb to 200 kb, preferably 0.5 kb to 100 kb. The sample of nucleic acids may also comprise hgDNA in the Mb size range.

After amplification, the amplified nucleic acids may be recovered from the support. This may be done by flushing the support with a sufficient amount of buffer. If the support is contained in a column, the amplified nucleic acids may be recovered from the support by elution from said column. The principles of elution are well-known from chromatography.

In certain cases it might be convenient if the support is removed from aqueous solutions after contacting the support with some or all of the reagents for carrying out amplification. For example, the support is first contacted with the desired reaction buffer including nucleotides and primer. The support may then be shortly spun dry in order to allow new fluid to enter the support. A small volume containing the reaction buffer, sample of nucleic acids, and the polymerase may be added to the support and allowed to enter the interspaces. Afterwards, the support may be put in an incubator for a desired time and temperature for amplification. After the amplification process the sample may retrieved by addition of an elution buffer and/or a subsequent centrifugation. Accordingly, in one aspect of the method the support is partly or completely dried between the step of contacting the support with reagents for carrying out an amplification reaction and contacting the support with a sample of nucleic acids. In another aspect of the method the support is partly or completely dried between the steps of carrying out the nucleic acid amplification reaction in the first cavities of the support and recovering the amplified nucleic acids from the support. The step of the second aspect may be also carried out in addition to the step of the first aspect. Drying may be achieved by centrifugation, aspiration, vacuum, heat or lyophilisation.

In a certain aspect of the invention, dry and non-permeable beads can be used for a batch type setup in conventional reaction tubes. In this case either the ready mix of buffer, sample of nucleic acids, nucleotides, and polymerase is added to a reaction tube containing the dry beads, or vice versa. The reaction tube can be transferred into an incubator or thermocycler to perform the amplification under desired conditions. After the amplification step, the sample is retrieved by transferring the entire tube content into an empty spin column. The elution is performed by adding elution buffer as described above. Another way to retrieve the amplified sample without spin columns is to add excess elution buffer into reaction tube, thereby re-suspending the beads and releasing enclosed amplicons. The beads-free supernatant containing the amplified nucleic acids can be used either directly or concentrated and/or purified for further analysis if desired.

### Support, kit and its uses

The invention further relates to a support as described in the context of the method above or in the following. In particular, the invention relates to a support comprising a plurality of interconnected first cavities, wherein all or at least a fraction of the interconnections reduce or prevent a convective flow between the first cavities during an nucleic acid amplification reaction, wherein the support further comprises a plurality of second cavities communicating with the first cavities, and wherein the second cavities serve as a repository for and comprise all or at least a fraction of reagents for carrying out an amplification reaction.

Preferably, the support is dry or wherein at least the reagents for carrying out the amplification reaction are dry. For example, the support is contacted with all or at least a fraction of the reagents for carrying out an amplification reaction, and, afterwards, the support is dried, e.g. by lyophilisation. Preferably, the support is contained within a column as described above.

The invention further relates to a kit comprising a support as described herein, a polymerase, and optionally the following: nucleotides and/or primers. As used herein, a "kit" is a packaged combination optionally including instructions for use of the combination and/or other reactions and components for such use.

One possible kit for isothermal amplification can be realised by providing a centrifuge spin column which is packed with a support as described herein. In case of permeable beads (e.g. a gel-filtration material), the column is preferably pre-equilibrated with the desired reaction buffer including nucleotides and primer. Most preferably, the support is provided in dry form.

The invention also relates to the use of a support as defined herein or use of the above kit for nucleic acid amplification, preferably, for whole genome amplification.

### FIGURE CAPTIONS

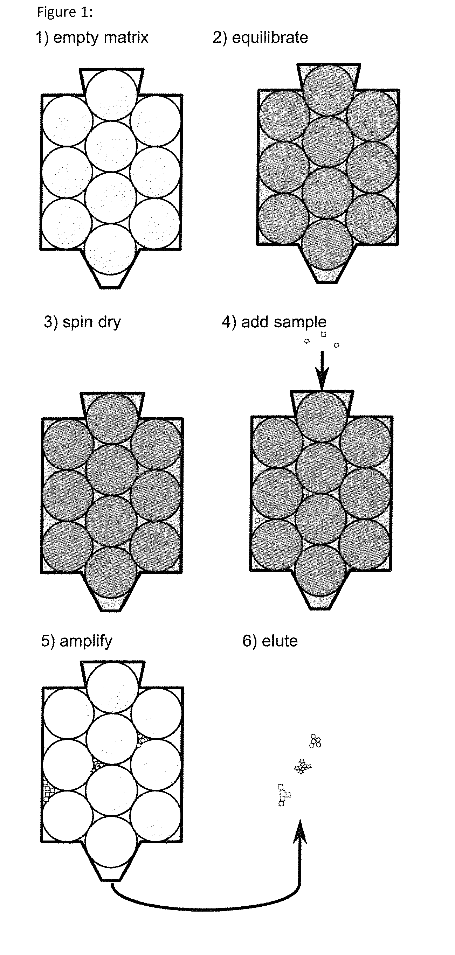

Figure 1: Principle of an interspacial amplification method with repository according to a preferred embodiment of the present method. 1) empty support; 2) contacting with reagents for amplification. Reagents are soaked into the beads; 3) optional step: spinning dry of the support. Reagents remain in the beads; 4) contacting the support with sample comprising nucleic acids (in this example 3 different nucleic acids). The nucleic acids distribute in different cavities; 5) nucleic acid amplification in individual cavitites; 6) recovering the amplified nucleic acids.

### EXAMPLES

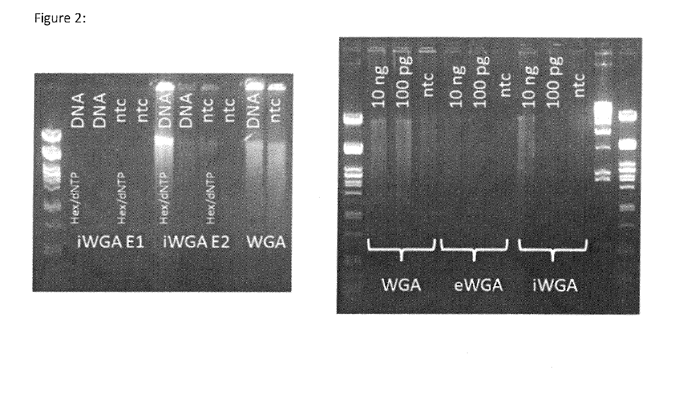

Whole-genome amplification (WGA) was performed in a matrix support prepared from Sephadex G-50 fine. The reaction mixture consisting of Phi29 DNA polymerase based amplification including template DNA (human genomic DNA) were directly applied to pre-equilibrated Sephadex G-50 fine matrix. Sepharose iWGA (interstitial WGA) reactions were carried out according to the following protocol: WGA reactions were prepared according to the "illustra GenomiPhi V2 DNA Amplification Kit" (GE Healthcare Life Sciences) containing 10 ng human genomic DNA in 10 µL reaction volumes. Before addition of the WGA reaction mixture on top of the Sepahdex G-50 fine matrix, the Sephadex G-50 fine matrix was pre-equilibrated according to the following protocol: from a Sephadex G-50 fine solution (1 g Sephadex G-50 fine in 16 mL ddH2O) 200 µL were transferred into a spin column (Zymo DNA Clean & Concentrator) and centrifuged (3000 g for 30 s). Resuspension and washing of Sephadex G-50 fine was performed by addition of 200 µL 1x Phi29 reaction buffer (NEB) followed by centrifugation (3000 g for 30 s). Pre-equilibration was performed with the addition of 50 µL 1x Phi29 reaction buffer (NEB) containing 1 µM random hexamers and 10 mM dNTPs. After incubation for 15 min and centrifugation (3000 g for 30 s) the WGA reaction mixture was applied. Incubation of the reaction was performed for 16 hours at 30° C, evaporation of the immersed WGA reaction mixture was prevented by sealing the columns containing the Sephadex G-50 fine matrix with adhesive tape. Recovery of the amplified product was performed by the addition of 30 µL 10 mM Tris-Cl, pH 8.5 followed by centrifugation (10000 g for 30 s). Visualization of reaction products was performed by agarose gel electrophoresis (Figure 2).

## Claims

1. A nucleic acid amplification method comprising the steps of:
a. providing a support comprising a plurality of interconnected first cavities;
b. contacting said support with reagents for carrying out an amplification reaction;
c. contacting said support with a sample of nucleic acids; and
d. carrying out the nucleic acid amplification reaction in said first cavities;
wherein all or at least a fraction of the interconnections reduce or prevent a convective flow between the first cavities during amplification.

2. The nucleic acid amplification method of claim 1, wherein the support is made of a porous material or a plurality of beads or a combination thereof.

3. The nucleic acid amplification method of any one of the preceding claims, wherein the support further comprises a plurality of second cavities communicating with the first cavities, and wherein the second cavities serve as a repository for the reagents for carrying out an amplification reaction.

4. The nucleic acid amplification method of claim 3, wherein the support is made of porous beads, wherein the first cavities are formed by the interstitial void between the beads, and wherein the second cavities are formed by the pore system within the beads.

5. The nucleic acid amplification method of any one of the preceding claims, wherein the beads on average have a diameter of 1 µm to 1000 µm, preferably 2 µm to 200 µm, most preferably 5 µm to 100 µm.

6. The nucleic acid amplification method of any one of the preceding claims, wherein the support is made of glass, magnetite, polystyrene, an acrylate or a polysaccharide, preferably polyacrylamide, polymethacrylate, agarose, sepharose and dextran.

7. The nucleic acid amplification method of any one of the preceding claims, wherein the amplification reaction is a whole genome amplification method.

8. The nucleic acid amplification method of any one of the preceding claims, wherein the reagents for carrying out an amplification reaction comprise at least a polymerase, nucleotides and one or more primers, or a polymerase, nucleotides and a primase.

9. The nucleic acid amplification method of claim 8, wherein the primers are selected from the group of random primers, degenerate primers and random exonuclease-resistant primers, and/or wherein the polymerase is selected from the group of Phi 29 polymerase.

10. The nucleic acid amplification method of any one of the preceding claims, wherein the support is contained in a column having openings at the top and the bottom, and wherein the reagents and the sample is contacted with the support by loading said reagents and said sample, respectively, onto said column via the top or bottom opening.

11. The nucleic acid amplification method of claim 10, wherein the amplified nucleic acids are recovered from the support by elution from said column.

12. A support comprising a plurality of interconnected first cavities, wherein all or at least a fraction of the interconnections reduce or prevent a convective flow between the first cavities during an nucleic acid amplification reaction, wherein the support further comprises a plurality of second cavities communicating with the first cavities, and wherein the second cavities serve as a repository for and comprise reagents for carrying out an amplification reaction.

13. The support of claim 12, wherein the support is dry or wherein at least the reagents for carrying out the amplification reaction are dry.

14. Kit comprising a support as defined in the previous claims 1 to 6, 12 or 13, a polymerase, and optionally the following: nucleotides and/or primers.

15. Use of a support as defined in the preceding claims 1 to 6, 12 or 13 or use of a kit according to claim 14 for nucleic acid amplification, preferably, for whole genome amplification.
